# EUROPEAN PATENT SPECIFICATION

(11) **EP 4 522 042 B1**
(45) Date of publication and mention of the grant of the patent: **04.11.2026**
(21) Application number: 22729077.2
(22) Date of filing: 11.05.2022
(51) Int. Cl.: A61B 17/22

(54) **ASPIRATION SYSTEM CONFIGURED FOR ASPIRATION OF VASCULAR DEBRIS FROM THE VASCULATURE**
ASPIRATIONSSYSTEM ZUR ASPIRATION VON GEFÄSSFRAGMENTE AUS DEM GEFÄSSSYSTEM
SYSTÈME D'ASPIRATION CONFIGURÉ EN VUE DE L'ASPIRATION DE DÉBRIS VASCULAIRES À PARTIR DU SYSTÈME VASCULAIRE

(43) Date of publication of application: 19.03.2025
(73) Proprietor: Straub Medical AG, 7323 Wangs (CH)
(72) Inventor: LAVRIJSEN, Tom, 7323 Wangs (CH); BAHNMUELLER, Bruno, 7323 Wangs (CH)
(74) Representative: Hoffmann Eitle
(86) International application number: PCT/EP2022/062748
(87) International publication number: WO 2023/217362

(56) References cited:
- US-A1- 2020 008 840
- US-A1- 2020 009 301
- US-A1- 2020 297 362
- US-B2- 10 441 460

## Description

### Technical Field

The present disclosure relates to an aspiration system configured for aspiration of vascular debris from the vasculature. Also, the present disclosure relates to a control unit for controlling aspiration using an aspiration catheter as well as a method for aspiration.

### Background

For vascular treatment, in particular removal of occlusions within a blood vessel, aspiration systems are known. In addition to aspiration, further treatments may be added, such as based on thrombectomy or atherectomy.

It is known from US 2015/0342682 A1 that suction provided by a proximal vacuum pump is used to suck in material at a distal end of a catheter. Usually, at the distal end, the catheter has a port serving as an opening for receiving material to be removed from the vasculature.

Pulsing characteristics can be used in connection with aspiration. As such, it is known to utilise a series of constant and/or varying pressure pulses at such port, as this is beneficial in aspirating vascular debris.

In other prior art systems, not only a single port for receiving the vascular debris, but also a second port may be provided.

US 2020/009301 A1 relates to an aspiration catheter that is provided including a proximal section and a distal section extendable through the proximal section. A vacuum transfer tool may be coupled to a proximal end of the proximal section. The vacuum transfer tool may include a proximal transfer tube and a distal transfer tube, each having an aspiration port in communication with a vacuum source. The proximal transfer tube may be removably received within a proximal end of the distal transfer tube. The distal section of the catheter may be inserted into the proximal section through the proximal transfer tube. The proximal transfer tube may maintain a vacuum around the proximal end of the distal section of the catheter such that when the distal section is removed by decoupling the proximal and distal transfer tubes, the vacuum within the distal transfer tube and proximal section of the catheter is maintained, preventing the escape of any clots. US 2020/008840 A1 is further prior art.

### Summary

Claims 1 and 15 define the invention and dependent claims disclose embodiments. No surgical methods are claimed per se. The present disclosure may support improvement of aspiration and/or removal of vascular debris from blood vessels.

An aspiration system is configured for aspiration of vascular debris from the vasculature, wherein the system comprises a catheter comprising at least one or two lumens configured for accommodating vascular debris and transporting vascular debris toward a proximal end of the catheter by way of suction, and at least first and second ports in fluid communication with the lumen(s) and for entry of vascular debris from the vasculature into the lumens. The first and second ports are located at a distal part of the catheter and are distanced from each other. The system is configured to facilitate aspiration of vascular debris by agitating vascular debris by providing coordinated pressure pulses, optionally suction pulses, at the first and second ports.

Pressure pulses provide discontinuous pressure levels or pressure fluctuations, such that the pressure changes over time. Optionally, the pulses may be periodic. Pressure pulses may be regarded as pressure difference pulses, as the pressure is increased or decreased relative to the ambient pressure. Pressure pulses are applied to the first and second ports in a coordinated way. Optionally, the pressure pulses may be suction pulses. This means that the pressure pulses may be based on negative pressure compared to the ambient pressure by way of suction. However, also the alternative, namely pressure pulses based on increased pressure relative to the ambient pressure, is conceivable.

Coordination of pressure pulses means that the pressure pulses in connection with the first and second ports are not independently from each other or not individually set, but are coordinated relative to each other in that the combination, in particular temporal interaction, of the pulses at the first and second ports supports agitation and, hence, aspiration. Coordination is implemented such that aspiration of vascular debris by agitating the vascular debris by way of the pressure pulses applied at the first and second ports is improved. In particular, it is desired to bring vascular debris into movement (agitation) based on the timing of pressure pulses acting at the first and second ports.

Specifically, the movement of the vascular debris may be between the first and second ports, or more generally speaking, in the opposing directions along which the ports are aligned.

By agitating, i.e. imparting movement to, vascular debris, separation from the vessel wall of the vascular debris is supported, so that the vascular debris becomes loose, possibly also macerated, and can be smoothly sucked in the catheter. Also, it is conceivable that the present disclosure supports aspiration of emboli, i.e. of lose vascular debris, via one of the ports, in that, for example, an embolus may be broken up by way of opposite forces acting at the ports on it.

Generally, as the vascular debris is being sucked in along the lumen(s), suction does not need to be the only force acting on the vascular debris. For example, additional forces originating from a rotating helix inside the lumen may act on the vascular debris.

The ports may be located directly in the catheter tube, or may be indirectly part of the catheter in that the ports are provided in an entity positioned distal to the actual catheter tube, for example.

Optionally, the first and second ports may be positioned along the length direction of the catheter, i.e. the direction of propagation of the pressure wave (s). In other words, the ports may have a different length position in that the ports are located at different distances relative to the distal end of the catheter. As such, the first and second ports may be regarded as proximal and distal ports, respectively. It is, however, not excluded that the ports are at the same longitudinal position

Vascular debris may comprise clot, calcifications, emboli, thrombi, etc. in the vasculature.

Also contemplated is the use of the aspiration system of the present disclosure, and a method for aspirating vascular debris.

Optionally, the system is configured to facilitate reciprocating and/or oscillating movement of vascular debris by way of the pressure pulses and/or due to the arrangement of the locations of the first and second ports. Optionally, the pressure pulses can be alternated between the first and second ports. By initiating a reciprocating movement and/or oscillation of the vascular debris, loosening from the vasculature due to repeated force-exertion may be supported.

In a preferred embodiment, the system further comprises a control unit configured to impart to the catheter pressure pulses at the first and second ports, wherein the pressure pulses at the first port differ from the pressure pulses at the second port, optionally as to the wavelength, further optionally as to the phase.

By way of a selection of the pressure pulses, controlled by a control unit, agitation of the vascular debris can be optimized. The difference in connection with the pressure pulses can be based on the wavelength and/or the phase. As such, by selecting a wavelength for the pressure pulses at the first and second ports, the difference of the pressure pulses at the first and second ports can be chosen accordingly. One way to control the difference is by way of the wavelength, i.e. frequency, of the pressure pulses, and another is the phase of the wave of the pressure pulses relative to each other, i.e. the phase shift between the first and second pulses.

Optionally, the pressure pulses at the first and second ports are periodic and have first and second wavelengths, respectively. The first wavelength is 1.5 to 20, further optionally 2 to 5 times the second wavelength. As the differences in wavelengths are in this range, it is considered that the pulses exert forces onto the vascular debris that result in an appropriate movement and, hence, better removal from the vasculature.

The different wavelengths at the first and second ports may be provided by different vacuum pumps.

As such, different wavelengths may be provided by different pumps, meaning that the first port may be connected to a first pump, and the second port may be connected to a second pump. Specifically in this case, the first and second ports may be positioned at the same length, at different radial positions, i.e. different positions on the circumference of the catheter.

If pressure pulses at the first and second ports are periodic and have the same wavelength, the phase shift between the pressure pulses at the first port relative to the pressure pulses at the second port may substantially fulfil 0.2 to 0.8 times the wavelength. Optionally, the phase shift is 0.2 to 0.6 times the wavelength, further optionally corresponds to one half of the wavelength. In this context, it is noted that it is not required for the pressure pulses to be completely out of phase, but that a non-matching phase may be considered as sufficient, as it already ensures that the pressure difference at one port is higher than the pressure difference at the other report, in one moment in time, and vice versa, so that a periodic change of the prevailing pressure difference leads to a reciprocating movement of the vascular debris in the region of or between the ports.

The phase shift does not need to be constant during a procedure. It is conceivable that the phase shift between the pressure pulses at the first and second ports changes over time. As such, a time-dependent or varying phase shift is generally contemplated. For example, the phase shift may approach or converge towards 0.5 times the wavelength over time during a procedure.

Alternatively or additionally, the wavelength(s) do not need to be constant during a procedure. It is conceivable that the difference in wavelengths (or wavelength ratio) between the pressure pulses at the first and second ports changes over time. As such, a time-dependent or varying wavelength-difference is generally contemplated. For example, the wavelength difference may approach or converge towards 2 over time during a procedure.

In some embodiments, one of the at least two lumens is referred to as first lumen and in fluid connection with the first port, but not in fluid communication with the second port. The other one of the at least two lumens is referred to as second lumen and in fluid communication with the second port, but not in fluid communication with the first port. This may be reflected in embodiments, in which different wavelengths of pressure pulses are provided by different pumps, so that the first pump is connected via the first lumen to the first port, wherein the second pump connects via the second lumen to the second port.

Generally, the first and second lumens may be concentric or in a D-shaped configuration.

Optionally, the catheter comprises a first connecting element for connecting the first lumen to a first pressure system, and a second connecting element for connecting the second lumen to a second pressure system different to the first pressure system. As such, the catheter has separate lumens and allows for connection to a respective pump.

The system may comprise a pressure control unit configured to impart to the catheter pressure pulses at the first and second ports, wherein a phase shift of the pressure pulses applied to the first lumen relative to the pressure pulses applied to the second lumen fulfils 0.2 to 0.8 times the wavelength, optionally 0. 4 to 0.6 times the wavelength, further optionally 0.5 times the wavelength, at the first and second ports. As such, it is the pressure control unit, optionally of the pump, which provides for the phase shift at the different ports.

Optionally, this phase shift may be present at the first connecting element and the second connecting element. This means that the phase shift is applied at the first and second connecting elements, i.e. by the pressure control units at the proximal end of the lumens.

In an embodiment, a first connecting element for connecting the first lumen to a common pressure system, and a second connecting element for connecting the second lumen to the common pressure system are provided. The first and second connecting elements are optionally united and the system further optionally comprises the common pressure system. Accordingly, the catheter may have separate lumens, but the catheter is connected to the same pressure system, e.g. the same pump. As such, the pressure difference between the first and second ports is not based on the pressure system, which is the same pressure system, but the pressure difference is imparted downstream of the pumps.

Alternatively, when the first and second ports are in fluid communication with a common lumen representing the at least one lumen, the system further optionally comprises a common pressure system for supplying the pressure pulses to the common lumen. In such embodiment, a single lumen for connecting the same pressure system with both ports is provided.

In one embodiment, a switch is provided in the common lumen configured for alternatingly switching fluid communication between an open state between the common lumen and the first port, on the one hand, and a substantially closed state between the common lumen and the second port, on the other hand. And vice versa, namely between an open state between the common lumen and the second port, on the one hand, and a substantially closed state between the common lumen and the first port, on the other hand. In other words, the switch switches between a first state characterized by an open state facilitating fluid communication between the common lumen and the first port, and a substantially closed state in connection with the common lumen and the second port, and a second state characterized by an open state facilitating fluid communication between the common lumen and the second port, and a substantially closed state in connection with the common lumen and the first port. The respective closed state includes a reduction of the fluid communication of at least 50%, optionally at least 30%, or optionally at least 20%, compared to the respective open state. Optionally, a switching interval between the open and closed state fulfils 0.2 to 0.8 times the wavelength, optionally 0.4 to 0.6 times the wavelength, further optionally 0.5 times the wavelength. As such, the switch provides a pressure difference between the first and second ports, so that a prevailing force periodically acts on the vascular debris. The most powerful pressure difference is expected when the switching interval is half of the wavelength.

According to the present disclosure, more than one port is provided. More than two ports are conceivable. One lumen per port may be provided. Alternatively, the ports may share a common lumen.

Optionally, the catheter may comprise further ports in addition to the first and second ports. The further ports are optionally provided at different locations in the direction of propagation of the pressure pulses, optionally along the length direction of the catheter. As per the embodiments as disclosed above for two ports, the additional ports may have a respective lumen, so that optionally, one lumen per port is present in the catheter. Depending on the position relative to each other of the first, second, third, fourth, and so on ports, an appropriate phase shift is to be chosen, so as to support agitation of the vascular debris, in particular reciprocating movement thereof.

As to a control unit of the present disclosure, a control unit may be for controlling aspiration using an aspiration catheter, in particular the aspiration system of the present disclosure. The catheter of the control unit may comprise at least one or two lumens configured for accommodating vascular debris and transporting vascular debris toward a proximal end of the catheter by way of suction, at least first and second ports in fluid communication with the lumens and for entry of vascular debris from the vasculature into the lumens, wherein the first and second ports are located at a distal part of the catheter and are distanced from each other, wherein the control unit is configured to control coordinated application of pressure pulses at the first and second ports such that vascular debris is agitated.

Optionally, the control unit is configured to facilitate reciprocating movement of vascular debris by way of the pressure pulses, optionally by alternating pressure pulses between the first and second ports.

Optionally, the control unit is configured to control the catheter pressure pulses at the first and second ports such that the pressure pulses at the first port differ from the pressure pulses at the second port, optionally as to the wavelength, further optionally as to the phase.

Optionally, the pressure pulses at the first and second ports are periodic and have first and second wavelengths, respectively, wherein the first wavelength is 1.5 to 20, optionally 2 to 5 times the second wavelength.

The catheter may comprise one of the at least two lumens referred to as first lumen and in fluid communication with the first port, but not in fluid communication with the second port. The other one of the at least two lumens is referred to as second lumen and in fluid communication with the second port, but not in fluid communication with the first port. The control unit is configured to control the pressure pulses at the first and second ports such that a phase shift between the pressure pulses applied to the first lumen relative to the pressure pulses applied to the second lumen fulfils 0.2 to 0.8 times the wavelength, optionally 0.4 to 0.6 times the wavelength, further optionally 0.5 times the wavelength at the first and second ports.

Optionally, the catheter may comprise a first connecting element for connecting the first lumen to the first pressure system, and a second connecting lumen for connecting the second lumen to a second pressure system other than the first pressure system, wherein the phase shift is 0.2 to 0.8 times the wavelength, optionally 0.4 to 0.6 times the wavelength, further optionally 0.5 times the wavelength between the pressure pulses at the first connecting element and the pressure pulses at the second connecting element.

The present disclosure also relates to the method of aspirating vascular debris from the vascular system.

### Brief Description of the Drawings

- Fig. 1a: shows a cross-section of a vessel with a catheter positioned within the vessel.
- Fig. 1b: shows wavelengths according to the disclosure.
- Fig. 2a: shows a catheter having separate lumens.
- Fig. 2b: shows a proximal part of a catheter having two separate lumens and two pressure systems.
- Fig. 3: shows a catheter with a pressure system and a control unit, wherein the catheter has a switch.

### Detailed Description

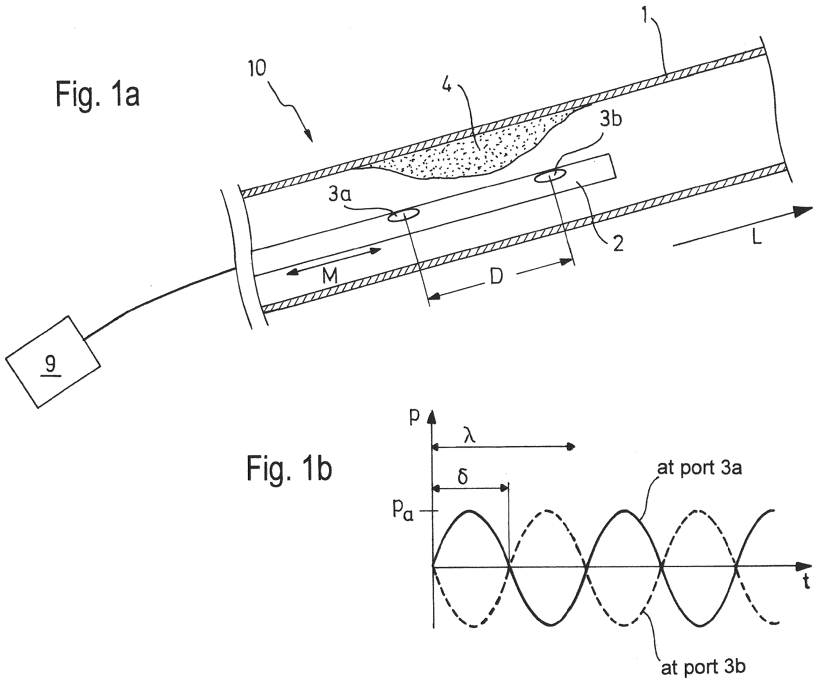

The present disclosure relates to an aspiration system 10 configured for aspiration of vascular debris 4 from the vasculature, i.e. the vessel walls 1. The system 10 comprises a catheter 2 comprising at least two lumens 5. The catheter 2 extends in the longitudinal direction L of the catheter. In Fig. 1a, first 3a and second 3b ports according to a first embodiment are shown. The lumen(s) 5 is/are configured for accommodating vascular debris 4 and transporting vascular debris 4 towards a proximal end of the catheter 2 by way of suction. At least the first 3a and second 3b ports are in fluid communication with the lumen 5 and allow for entry of vascular debris 4 from the vasculature 1 into the lumens 5.

The first 3a and second 3b ports are located at a distal part of the catheter 2 and are distanced from each other by a distance D. The system 10 is configured to facilitate aspiration of vascular debris 4 by agitating vascular debris 4 by providing coordinated pressure pulses at the first 3a and second 3b ports. The coordinated pressure pulses between the ports 3a and 3b act on the debris 4, which is, in the state shown in Fig. 1a, still connected to the vessel wall 1. Upon suction by way of the ports 3a and 3b and the coordinated pulses, the vascular debris 4 is moved and, due to the change of direction of the suction due to the coordinated pressure pulses at the ports 3a and 3b, a movement of the vascular debris along the direction of the arrow M is initiated. By way of this movement, loosening and separation of the vascular debris 4 from the vessel wall 1 is supported. In a subsequent state, not shown in the drawings, the vascular debris 4 would, as loosened emboli, be present directly between the ports 3a and 3b and would be sucked in via one of these port, or by both ports if the emboli is broken up, for example.

The system 10 allows for reciprocating movement, i.e. oscillating movement in the opposite directions along the arrow M of vascular debris 4 by way of the pressure pulses. Between the first 3a and second 3b ports, the pressure pulses may alternate, already because of the location of the first 3a and second 3b ports.

As shown in Fig. 1a, the system 10 further comprises a control unit 9 configured to impart to the catheter 2 pressure pulses at the first 3a and second 3b ports. The pressure pulses at the first port 3a differ from the pressure pulses at the second port 3b. In particular, the difference may be the wavelength and/or the phase.

The pressure pulses may be periodic. The pressure pulses may have first and second wavelengths λ, wherein the first wavelength may differ from the second wavelength. For example, the first wavelength may be 1.5 to 20, or to 2 to 5 times the second wavelength.

Fig. 1b reflects pressure pulses having the same wavelength λ. In this example, the wave is a sine wave. The phase shift δ between the pressure pulses at the first port 3a and the second port 3b is half of the wavelength λ. As such, the pressure pulses are exactly opposite to each other, which is expected to result in the largest pressure difference between the ports 3a and 3b in one moment in time and, as such, to impart the maximum movement onto the vascular debris 4. The phase shift δ may be constant during a procedure or may change over time, i.e. the phase shift may vary. Alternatively or additionally, it is conceivable that the difference in wavelength, i.e. the difference between the wavelength at the first port 3a and the wavelength at the second port 3b, varies over time. For example, while the wavelength at the first port 3a may be constant, the wavelength at the second port 3b may vary during a procedure, or vice versa, or both wavelengths may vary over time.

Fig. 1b indicates an amplitude of the wave corresponding to the maximum pulse pressure difference. The pressure pₐ is considered as the pressure of the ambient air. It is considered that the absolute amplitude of the pressure is of less relevance in connection with the present disclosure. The amplitude of the pressure pulses may differ between the first and second ports, i.e. between the first and second pressure pulses, or may be the same.

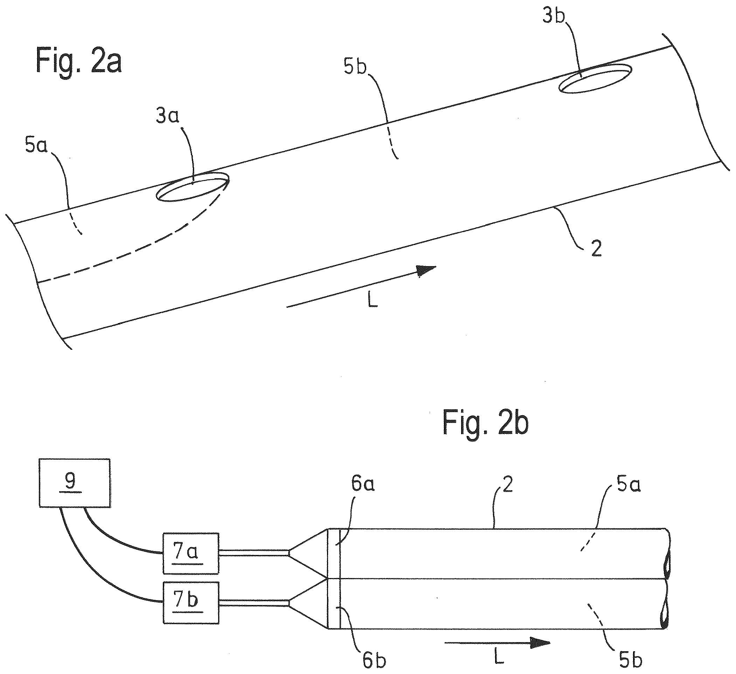

Fig. 2a shows a second embodiment having a catheter 2 having a first port 3a in fluid communication with the first lumen 5a. The first lumen 5a is not in fluid communication with the second port 3b. The second lumen 5b is in fluid communication with the second port 3b, but not in fluid communication with the first port 3a. The pressure pulses may be coordinated based on the distance D between the ports 3a and 3b in the length direction D. Alternatively or additionally, the following features may be realized:

In Fig. 2b, the catheter 2 further comprises a first connecting element 6a for connecting the first lumen 5a to a first pressure system 7a, and a second connecting element 6b for connecting the second lumen 5b to a second pressure system 7b other than the first pressure system 7a. A first connecting element 6a and a second connecting element 6b are provided. Hence, as shown in Fig. 2b, a first subsystem is formed by the first port 3a, the first lumen 5a and the first pressure system 7a, whilst a second subsystem is formed by the second port 3b, the second lumen 5b, and the second pressure system 7b.

A control unit 9 is configured to impart to the catheter 2 pressure pulses for the first 3a and second 3b ports. A phase shift between the pressure pulses applied to the first lumen 5a relative to the pressure pulses applied to the second lumen 5b is ideally half of the wavelength at the first 3a second 3b ports. The phase shift at the first 3a and second 3b ports may correspond to the pressure difference at the first connecting element 6a and the second connecting element 6b already. While Fig. 2b shows two different pressure system 7a and 7b, it is conceivable that a common pressure system 7 (not shown in the drawings) is used. As such, a first connecting element 6a for connecting the first lumen 5a to the common pressure system 7, and a second connecting element 6b for connecting the second lumen 5b to the common pressure system 7 may be provided. It is conceivable that the first and second connecting elements 6a and 6b are united and that the system 10 further comprises the common pressure system 7.

While the schematic drawing of Fig. 2b shows two lumens 5a and 5b positioned next to each other, it is contemplated to position the lumens 5a and 5b differently than next to each other. For example, the lumens 5a and 5b may be concentric or in a D-shaped arrangement (i.e. one lumen having a crescent shape and the other lumen having a corresponding shape, such as a circular shape substantially surrounded by the crescent).

At the proximal end of the catheter, a handle (not shown in the drawings) may be provided. The handle may be reusable, while only the catheter 2 is disposable. Alternatively, the handle may be disposable as well and may even include a pressure system, such as a pump, which is disposable.

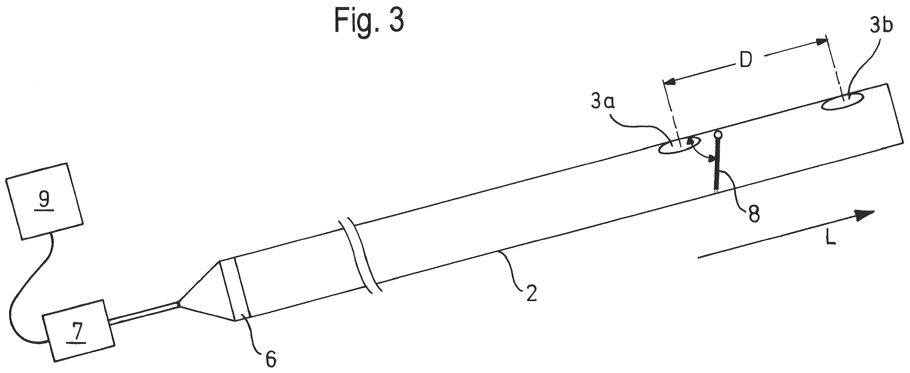

In a third embodiment, as shown in Fig. 3, a common lumen 5, a common connecting element 6, as well as a common pressure system 7 are provided. Within the catheter 2, towards the distal end, optionally downstream the first port 3a, but upstream the second port 3b, a switch 8 is provided. In an embodiment, the switch 8 may be a flap, which may be pivoted such that, in an open state, the lumen 5 connects with the second port 3b, whilst the first port 3a is closed, and a substantially closed state in connection with the first port 3a is achieved. The switching interval t between the open and closed state may, ideally, be half of the wavelength of the pressure pulses imparted by the pressure system 7. As such, (positive or negative) pressure is applied only via the first 3a or second 3b port. As indicated in Fig. 3, the distance between the first 3a and second 3b port is D and may correspond to approximately half of the wavelength. In this case, no switch 8 may be needed, when the distance D corresponds to half of the wavelength, for example.

Specifically, the distance D refers to the direction of propagation of the pressure pulses, in particular the length direction L of the catheter. The catheter is configured to provide the pressure pulses at the first 3a and second 3b ports, which pressure pulses are periodic and have the same wavelength.

Although the drawings show embodiments having first 3a and second 3b ports, also additional ports 3 may be provided. Further ports may be in between the first 3a and second 3b ports. Optionally, the additional ports are provided at different locations in the direction of propagation of the pressure pulses, in particular the length direction L of the catheter.

The present invention also refers to a control unit 9, as shown in the drawings, the control unit 9 configured to control coordinated application of pressure pulses at the first 3a and second 3b ports (or further ports) such that vascular debris is being agitated. The scope of the invention is defined by the claims.

### List of Reference Signs

- 1: vessel wall
- 2: catheter
- 3: port
- 4: vascular debris
- 5: lumen
- 6: connecting element
- 7: pressure system (pump)
- 8: switch
- 9: control unit
- 10: aspiration system

- L: longitudinal/length direction of catheter
- M: directions of movement
- D: distance

## Claims

1. An aspiration system (10) configured for aspiration of vascular debris (4) from the vasculature, the system comprising a catheter (2) comprising
at least one or two lumen(s) (5) configured for accommodating vascular debris (4) and transporting vascular debris (4) toward a proximal end of the catheter (2) by way of suction,
at least first and second ports (3) in fluid communication with the lumen(s) (5) and for entry of vascular debris (4) from the vasculature into the lumen(s) (5), wherein
the first and second ports (3) are located at a distal part of the catheter (2) and are distanced from each other, and
the system (10) is configured to facilitate aspiration of vascular debris (4) by agitating vascular debris (4) by providing coordinated pressure pulses, optionally suction pulses, at the first and second ports (3), wherein the system is configured to facilitate reciprocating and/or oscillating movement of vascular debris (4) by way of the pressure pulses by alternating pressure pulses between the first and second ports (3).

2. The system of claim 1, wherein the system is configured to facilitate reciprocating and/or oscillating movement of vascular debris (4) due to the positions of the locations of the first and second ports (3).

3. The system of claim 1 or 2, wherein the system further comprises a control unit (9) configured to impart to the catheter (2) pressure pulses at the first and second ports (3), wherein the pressure pulses at the first port(3a) differ from the pressure pulses at the second port (3b), optionally as to at least one of the wavelength and the phase

4. The system of claim 3, wherein the pressure pulses at the first and second ports (3) are periodic and have first and second wavelengths, respectively, wherein the first wavelength is 1.5 to 20, optionally 2 to 5 times the second wavelength.

5. The system of claim 1 or 2, wherein the system is configured to provide pressure pulses at the first and second ports (3), which pressure pulses are periodic and have the same wavelength λ, and a phase shift θ between the pressure pulses at the first port (3a) relative to the pressure pulses at the second port (3b) substantially fulfils θ = (0.2 to 0.8)*λ, optionally (0.4 to 0.6)*λ, further optionally 0.5*λ.

6. The system of any of the preceding claims, wherein
one of the at least two lumens is a first lumen (5a) and in fluid communication with the first port (3a), but not the second port (3b),
the other one of the at least two lumens is a second lumen (5b) and in fluid communication with the second port (3b), but not with the first port (3a).

7. The system of claim 6, the catheter (2) further comprising a first connecting element (6a) for connecting the first lumen (5a) to a first pressure system (7a) and a second connecting element (6b) for connecting the second lumen (5b) to a second pressure system (7b) different to the first pressure system (7a).

8. The system of claim 6 or 7, the system further comprising a control unit (9) configured to impart to the catheter (2) pressure pulses at the first and second ports (3), wherein a phase shift θ between the pressure pulses applied to the first lumen (5a) relative to the pressure pulses applied to the second lumen (5b) fulfils θ = (0.2 to 0.8)*λ, optionally (0.4 to 0.6)*λ, further optionally 0.5*λ, at the first and second ports.

9. System of claims 7 and 8, wherein the phase shift θ fulfils θ = (0.2 to 0.8)*λ, optionally (0.4 to 0.6)*λ, further optionally 0.5*λ, between the pressure pulses at the first connecting element (6a) and the pressure pulses at the second connecting element (6b).

10. The system of any of the preceding claims 1 to 6, wherein the catheter further comprises
a first connecting element (6a) for connecting a first lumen (5a) to a common pressure system (7), and a second connecting element (6b) for connecting a second lumen (5b) to said common pressure system (7), the first (6a) and second (6b) connecting elements optionally being united, and/or the system optionally comprising the common pressure system (7).

11. The system of any of the preceding claims 1 to 2 and 5, wherein the first (3a) and second (3b) ports are in fluid communication with a common lumen (5), the system optionally comprising a common pressure system (7) for supplying the pressure pulses to the common lumen (5).

12. The system of claim 11, wherein the catheter comprises
a switch (8) in the common lumen (5), the catheter configured for alternatingly switching between a first state **characterized by** an open state facilitating fluid communication between the common lumen (5) and the first port (3a), and a substantially closed state in connection with the common lumen (5) and the second port (3b), and a second state **characterized by** an open state facilitating fluid communication between the common lumen (5) and the second port (3b), and a substantially closed state in connection with the common lumen (5) and the first port (3a), wherein the respective closed state includes a reduction of the fluid communication of at least 50%, optionally at least 30%, more optionally at least 20%, compared to the respective open state, and wherein optionally
a switching interval t between the open and closed states fulfils t = (0.2 to 0.8)*λ, optionally (0.4 to 0.6)*λ, further optionally 0.5*λ.

13. The system of any of the preceding claims 1, 2, 5, and 12, wherein a distance D between the first (3a) and second (3b) ports fulfils D = (n+ [0.2 to 0.8])* λ, optionally (n+ [0.4 to 0.6])* λ, further optionally (n+0.5)*λ, n=0, 1, 2, 3..., wherein the distance D refers to the direction of propagation of the pressure pulses, optionally in the length direction of the catheter, the catheter (2) configured to provide pressure pulses at the first (3a) and second (3b) ports, which pressure pulses are periodic and have the same wavelength λ.

14. The system of any of the preceding claims, wherein the catheter (2) comprises further ports (3) in addition to the first and second ports, wherein the further ports are optionally provided at different locations in the direction of propagation of the pressure pulses, optionally in the length direction of the catheter (2).

15. A control unit (9) for controlling aspiration using an aspiration catheter, the catheter (2) comprising
at least one or two lumen(s) (5) configured for accommodating vascular debris (4) and transporting vascular debris (4) towards a proximal end of the catheter (2) by way of a pressure difference, optionally suction,
at least first and second ports (3) in fluid communication with the lumen(s) (5) and for entry of vascular debris (4) from the vasculature into the lumen(s), wherein
the first and second ports (3) are located at a distal part of the catheter (2) and are distanced from each other, wherein
the control unit (9) is configured to control coordinated application of pressure pulses at the first and second ports (3) such that vascular debris is agitated, wherein the control unit (9) is configured to facilitate reciprocating and/or oscillating movement of vascular debris (4) by way of the pressure pulses by alternating pressure pulses between the first and second ports (3).

## Patentansprüche

1. Aspirationssystem (10), ausgebildet zur Aspiration von Gefäßfragmenten (4) aus dem Gefäßsystem, wobei das System einen Katheter (2) umfasst, umfassend
mindestens ein oder zwei Lumen (5), das/die zum Aufnehmen von Gefäßfragmenten (4) und zum Transportieren von Gefäßfragmenten (4) in Richtung eines proximalen Endes des Katheters (2) mittels Saugwirkung ausgebildet ist/sind,
mindestens einen ersten und einen zweiten Anschluss (3), die mit dem/den Lumen (5) in Fluidverbindung stehen und zum Eintritt von Gefäßfragmenten (4) aus dem Gefäßsystem in das/die Lumen (5) vorgesehen sind, wobei
der erste und der zweite Anschluss (3) sich an einem distalen Teil des Katheters (2) befinden und voneinander beabstandet sind, und
das System (10) dazu ausgebildet ist, Aspiration von Gefäßfragmenten (4) zu erleichtern, indem Gefäßfragmente (4) in Bewegung versetzt werden, indem koordinierte Druckimpulse, optional Saugimpulse, an dem ersten und dem zweiten Anschluss (3) bereitgestellt werden, wobei das System dazu ausgebildet ist, eine Hin- und Herbewegung und/oder eine oszillierende Bewegung von Gefäßfragmenten (4) mittels der Druckimpulse zu erleichtern, indem Druckimpulse zwischen dem ersten und dem zweiten Anschluss (3) abgewechselt werden.

2. System nach Anspruch 1, wobei das System dazu ausgebildet ist, eine Hin- und Herbewegung und/oder eine oszillierende Bewegung von Gefäßfragmenten (4) aufgrund der Positionen der Orte des ersten und des zweiten Anschlusses (3) zu erleichtern.

3. System nach Anspruch 1 oder 2, wobei das System weiter eine Steuereinheit (9) umfasst, die dazu ausgebildet ist, den Katheter (2) an dem ersten und dem zweiten Anschluss (3) mit Druckimpulsen zu beaufschlagen, wobei sich die Druckimpulse an dem ersten Anschluss (3a) von den Druckimpulsen an dem zweiten Anschluss (3b) unterscheiden, optional hinsichtlich mindestens einem von der Wellenlänge und der Phase

4. System nach Anspruch 3, wobei die Druckimpulse an dem ersten und dem zweiten Anschluss (3) periodisch sind und eine erste bzw. eine zweite Wellenlänge aufweisen, wobei die erste Wellenlänge das 1,5- bis 20-fache, optional das 2- bis 5-fache der zweiten Wellenlänge beträgt.

5. System nach Anspruch 1 oder 2, wobei das System dazu ausgebildet ist, Druckimpulse an dem ersten und dem zweiten Anschluss (3) bereitzustellen, wobei die Druckimpulse periodisch sind und dieselbe Wellenlänge λ aufweisen, und wobei eine Phasenverschiebung θ zwischen den Druckimpulsen an dem ersten Anschluss (3a) relativ zu den Druckimpulsen an dem zweiten Anschluss (3b) im Wesentlichen θ = (0,2 bis 0,8)*λ, optional (0,4 bis 0,6)*λ, weiter optional 0,5*λ erfüllt.

6. System nach einem der vorstehenden Ansprüche, wobei
eines der mindestens zwei Lumen ein erstes Lumen (5a) ist und mit dem ersten Anschluss (3a) in Fluidverbindung steht, jedoch nicht mit dem zweiten Anschluss (3b),
das andere der mindestens zwei Lumen ein zweites Lumen (5b) ist und mit dem zweiten Anschluss (3b) in Fluidverbindung steht, jedoch nicht mit dem ersten Anschluss (3a).

7. System nach Anspruch 6, wobei der Katheter (2) weiter ein erstes Verbindungselement (6a) zum Verbinden des ersten Lumens (5a) mit einem ersten Drucksystem (7a) und ein zweites Verbindungselement (6b) zum Verbinden des zweiten Lumens (5b) mit einem zweiten Drucksystem (7b), das sich von dem ersten Drucksystem (7a) unterscheidet, umfasst.

8. System nach Anspruch 6 oder 7, wobei das System weiter eine Steuereinheit (9) umfasst, die dazu ausgebildet ist, den Katheter (2) an dem ersten und dem zweiten Anschluss (3) mit Druckimpulsen zu beaufschlagen, wobei eine Phasenverschiebung θ zwischen den am ersten Lumen (5a) angewendeten Druckimpulsen relativ zu den am zweiten Lumen (5b) angewendeten Druckimpulsen θ = (0,2 bis 0,8)*λ, optional (0,4 bis 0,6)*λ, weiter optional 0,5*λ an dem ersten und dem zweiten Anschluss erfüllt.

9. System nach den Ansprüchen 7 und 8, wobei die Phasenverschiebung θ zwischen den Druckimpulsen an dem ersten Verbindungselement (6a) und den Druckimpulsen an dem zweiten Verbindungselement (6b) θ = (0,2 bis 0,8)*λ, optional (0,4 bis 0,6)*λ, weiter optional 0,5*λ erfüllt.

10. System nach einem der vorstehenden Ansprüche 1 bis 6, wobei der Katheter weiter Folgendes umfasst
ein erstes Verbindungselement (6a) zum Verbinden eines ersten Lumens (5a) mit einem gemeinsamen Drucksystem (7) und ein zweites Verbindungselement (6b) zum Verbinden eines zweiten Lumens (5b) mit dem gemeinsamen Drucksystem (7), wobei das erste (6a) und das zweite (6b) Verbindungselement optional vereinigt sind, und/oder wobei das System optional das gemeinsame Drucksystem (7) umfasst.

11. System nach einem der vorstehenden Ansprüche 1 bis 2 und 5, wobei der erste (3a) und der zweite (3b) Anschluss mit einem gemeinsamen Lumen (5) in Fluidverbindung stehen, wobei das System optional ein gemeinsames Drucksystem (7) zum Zuführen der Druckimpulse zu dem gemeinsamen Lumen (5) umfasst.

12. System nach Anspruch 11, wobei der Katheter Folgendes umfasst
einen Schalter (8) in dem gemeinsamen Lumen (5), wobei der Katheter zum abwechselnden Umschalten zwischen einem ersten Zustand, der durch einen offenen Zustand, der eine Fluidverbindung zwischen dem gemeinsamen Lumen (5) und dem ersten Anschluss (3a) erleichtert, und einen im Wesentlichen geschlossenen Zustand in Verbindung mit dem gemeinsamen Lumen (5) und dem zweiten Anschluss (3b) gekennzeichnet ist, und einem zweiten Zustand, der durch einen offenen Zustand, der eine Fluidverbindung zwischen dem gemeinsamen Lumen (5) und dem zweiten Anschluss (3b) erleichtert, und einen im Wesentlichen geschlossenen Zustand in Verbindung mit dem gemeinsamen Lumen (5) und dem ersten Anschluss (3a) gekennzeichnet ist, ausgebildet ist, wobei der jeweilige geschlossene Zustand eine Verringerung der Fluidverbindung um mindestens 50 %, optional mindestens 30 %, weiter optional mindestens 20 % im Vergleich zu dem jeweiligen offenen Zustand einschließt, und wobei optional
ein Schaltintervall t zwischen den offenen und den geschlossenen Zuständen t = (0,2 bis 0,8)*λ, optional (0,4 bis 0,6)*λ, weiter optional 0,5*λ erfüllt.

13. System nach einem der vorstehenden Ansprüche 1, 2, 5 und 12, wobei ein Abstand D zwischen dem ersten (3a) und dem zweiten (3b) Anschluss D = (n+ [0,2 bis 0,8])*λ, optional (n+ [0,4 bis 0,6])*λ, weiter optional (n+0,5)*λ, n=0, 1, 2, 3... erfüllt, wobei sich der Abstand D auf die Ausbreitungsrichtung der Druckimpulse bezieht, optional in der Längsrichtung des Katheters, wobei der Katheter (2) dazu ausgebildet ist, Druckimpulse an dem ersten (3a) und dem zweiten (3b) Anschluss bereitzustellen, wobei die Druckimpulse periodisch sind und dieselbe Wellenlänge λ aufweisen.

14. System nach einem der vorstehenden Ansprüche, wobei der Katheter (2) zusätzlich zu dem ersten und dem zweiten Anschluss weitere Anschlüsse (3) umfasst, wobei die weiteren Anschlüsse optional an unterschiedlichen Orten in der Ausbreitungsrichtung der Druckimpulse, optional in der Längenrichtung des Katheters (2), bereitgestellt sind.

15. Steuereinheit (9) zum Steuern von Aspiration unter Verwendung eines Aspirationskatheters, der Katheter (2) umfassend
mindestens ein oder zwei Lumen (5), das/die zum Aufnehmen von Gefäßfragmenten (4) und zum Transportieren von Gefäßfragmenten (4) in Richtung eines proximalen Endes des Katheters (2) mittels einer Druckdifferenz, optional Saugwirkung, ausgebildet ist/sind,
mindestens einen ersten und einen zweiten Anschluss (3), die mit dem/den Lumen (5) in Fluidverbindung stehen und zum Eintritt von Gefäßfragmenten (4) aus dem Gefäßsystem in das/die Lumen vorgesehen sind, wobei
der erste und der zweite Anschluss (3) sich an einem distalen Teil des Katheters (2) befinden und voneinander beabstandet sind, wobei
die Steuereinheit (9) dazu ausgebildet ist, eine koordinierte Anwendung von Druckimpulsen an dem ersten und dem zweiten Anschluss (3) so zu steuern, dass Gefäßfragmente in Bewegung versetzt werden, wobei die Steuereinheit (9) dazu ausgebildet ist, eine Hin- und Herbewegung und/oder eine oszillierende Bewegung von Gefäßfragmenten (4) mittels der Druckimpulse zu erleichtern, indem Druckimpulse zwischen dem ersten und dem zweiten Anschluss (3) abgewechselt werden.

## Revendications

1. Système d'aspiration (10) configuré pour l'aspiration de débris vasculaires (4) à partir du système vasculaire, le système comprenant un cathéter (2) comprenant
au moins une ou deux lumières (5) configurées pour recevoir des débris vasculaires (4) et transporter des débris vasculaires (4) vers une extrémité proximale du cathéter (2) par aspiration,
au moins des premier et second orifices (3) en communication fluidique avec la/les lumière(s) (5) et destinés à permettre l'entrée de débris vasculaires (4) à partir du système vasculaire dans la/les lumière(s) (5), dans lequel
les premier et second orifices (3) sont situés au niveau d'une partie distale du cathéter (2) et sont espacés l'un de l'autre, et
le système (10) est configuré pour faciliter l'aspiration de débris vasculaires (4) en agitant des débris vasculaires (4) par fourniture d'impulsions de pression coordonnées, facultativement d'impulsions d'aspiration, aux premier et second orifices (3), dans lequel le système est configuré pour faciliter un mouvement alternatif et/ou oscillatoire de débris vasculaires (4) au moyen des impulsions de pression en alternant les impulsions de pression entre les premier et second orifices (3).

2. Système selon la revendication 1, dans lequel le système est configuré pour faciliter un mouvement alternatif et/ou oscillatoire de débris vasculaires (4) en raison des positions des emplacements des premier et second orifices (3).

3. Système selon la revendication 1 ou la revendication 2, dans lequel le système comprend en outre une unité de commande (9) configurée pour communiquer au cathéter (2) des impulsions de pression au niveau des premier et second orifices (3), dans lequel les impulsions de pression au premier orifice (3a) diffèrent des impulsions de pression au second orifice (3b), facultativement quant à au moins une de la longueur d'onde et de la phase

4. Système selon la revendication 3, dans lequel les impulsions de pression au niveau des premier et second orifices (3) sont périodiques et présentent respectivement des première et seconde longueurs d'onde, dans lequel la première longueur d'onde est de 1,5 à 20, facultativement de 2 à 5 fois la seconde longueur d'onde.

5. Système selon la revendication 1 ou la revendication 2, dans lequel le système est configuré pour fournir des impulsions de pression aux premier et second orifices (3), lesquelles impulsions de pression sont périodiques et présentent la même longueur d'onde λ, et un déphasage θ entre les impulsions de pression au premier orifice (3a) par rapport aux impulsions de pression au second orifice (3b) satisfait sensiblement à θ = (0,2 à 0,8)*λ, facultativement (0,4 à 0,6)*λ, facultativement encore 0,5*λ.

6. Système selon l'une quelconque des revendications précédentes, dans lequel
l'une des au moins deux lumières est une première lumière (5a) et en communication fluidique avec le premier orifice (3a), mais pas avec le second orifice (3b),
l'autre des au moins deux lumières est une seconde lumière (5b) et en communication fluidique avec le second orifice (3b), mais pas avec le premier orifice (3a).

7. Système selon la revendication 6, le cathéter (2) comprenant en outre un premier élément de raccordement (6a) pour raccorder la première lumière (5a) à un premier système de pression (7a) et un second élément de raccordement (6b) pour raccorder la seconde lumière (5b) à un second système de pression (7b) différent du premier système de pression (7a).

8. Système selon la revendication 6 ou la revendication 7, le système comprenant en outre une unité de commande (9) configurée pour communiquer au cathéter (2) des impulsions de pression au niveau des premier et second orifices (3), dans lequel un déphasage θ entre les impulsions de pression appliquées à la première lumière (5a) par rapport aux impulsions de pression appliquées à la seconde lumière (5b) satisfait à θ = (0,2 à 0,8)*λ, facultativement (0,4 à 0,6)*λ, facultativement encore 0,5*λ au niveau des premier et second orifices.

9. Système selon les revendications 7 et 8, dans lequel le déphasage θ satisfait à θ = (0,2 à 0,8)*λ, facultativement (0,4 à 0,6)*λ, facultativement encore 0,5*λ entre les impulsions de pression au premier élément de raccordement (6a) et les impulsions de pression au second élément de raccordement (6b).

10. Système selon l'une quelconque des revendications 1 à 6, dans lequel le cathéter comprend en outre
un premier élément de raccordement (6a) pour raccorder une première lumière (5a) à un système de pression commun (7), et un second élément de raccordement (6b) pour raccorder une seconde lumière (5b) audit système de pression commun (7), le premier élément de raccordement (6a) et le second élément de raccordement (6b) étant facultativement réunis, et/ou le système comprenant facultativement le système de pression commun (7).

11. Système selon l'une quelconque des revendications 1 à 2 et 5 précédentes, dans lequel le premier orifice (3a) et le second orifice (3b) sont en communication fluidique avec une lumière commune (5), le système comprenant facultativement un système de pression commun (7) pour fournir les impulsions de pression à la lumière commune (5).

12. Système selon la revendication 11, dans lequel le cathéter comprend
un commutateur (8) dans la lumière commune (5), le cathéter étant configuré pour commuter alternativement entre un premier état **caractérisé par** un état ouvert facilitant une communication fluidique entre la lumière commune (5) et le premier orifice (3a), et un état sensiblement fermé, en relation avec la lumière commune (5) et le second orifice (3b), et un second état **caractérisé par** un état ouvert facilitant une communication fluidique entre la lumière commune (5) et le second orifice (3b), et un état sensiblement fermé, en relation avec la lumière commune (5) et le premier orifice (3a), dans lequel l'état fermé respectif inclut une réduction de la communication fluidique d'au moins 50 %, facultativement d'au moins 30 %, plus facultativement d'au moins 20 %, par rapport à l'état ouvert respectif, et dans lequel facultativement
un intervalle de commutation t entre les états ouvert et fermé satisfait à t = (0,2 à 0,8)*λ, facultativement (0,4 à 0,6)*λ, facultativement encore 0,5*λ.

13. Système selon l'une quelconque des revendications 1, 2, 5 et 12 précédentes, dans lequel une distance D entre le premier orifice (3a) et le second orifice (3b) satisfait à D = (n+ [0,2 à 0,8])*λ, facultativement (n+ [0,4 à 0,6])*λ, facultativement encore (n+0,5)*λ, n = 0, 1, 2, 3..., dans lequel la distance D se rapporte à la direction de propagation des impulsions de pression, facultativement dans la direction longitudinale du cathéter, le cathéter (2) étant configuré pour fournir des impulsions de pression au premier orifice (3a) et au second orifice (3b), lesquelles impulsions de pression sont périodiques et présentent la même longueur d'onde λ.

14. Système selon l'une quelconque des revendications précédentes, dans lequel le cathéter (2) comprend des orifices supplémentaires (3) en plus des premier et second orifices, dans lequel les orifices supplémentaires sont facultativement disposés à différents emplacements dans la direction de propagation des impulsions de pression, facultativement dans la direction longitudinale du cathéter (2).

15. Unité de commande (9) pour commander une aspiration au moyen d'un cathéter d'aspiration, le cathéter (2) comprenant
au moins une ou deux lumières (5) configurées pour recevoir des débris vasculaires (4) et transporter des débris vasculaires (4) vers une extrémité proximale du cathéter (2) au moyen d'une différence de pression, facultativement par aspiration,
au moins des premier et second orifices (3) en communication fluidique avec la/les lumière(s) (5) et destinés à permettre l'entrée de débris vasculaires (4) à partir du système vasculaire dans la/les lumière(s), dans laquelle
les premier et second orifices (3) sont situés au niveau d'une partie distale du cathéter (2) et sont espacés l'un de l'autre, dans laquelle
l'unité de commande (9) est configurée pour commander une application coordonnée d'impulsions de pression au niveau des premier et second orifices (3) de telle sorte que des débris vasculaires soient agités, dans laquelle l'unité de commande (9) est configurée pour faciliter un mouvement alternatif et/ou oscillatoire de débris vasculaires (4) au moyen des impulsions de pression en alternant les impulsions de pression entre les premier et second orifices (3).
